# EUROPEAN PATENT SPECIFICATION

(11) **EP 2 768 226 B1**
(45) Date of publication and mention of the grant of the patent: **31.07.2019**
(21) Application number: 12840320.1
(22) Date of filing: 12.10.2012
(51) Int. Cl.: H04N 13/00

(54) **3D ENDOSCOPE DEVICE**
3D-ENDOSKOPVORRICHTUNG
DISPOSITIF D'ENDOSCOPE 3D

(30) Priority: 14.10.2011 JP 2011226756
(43) Date of publication of application: 20.08.2014
(73) Proprietor: Olympus Corporation, Tokyo 192-8507 (JP)
(72) Inventor: NISHIMURA, Hisashi, Hachioji-shi, Tokyo 192-8507 (JP)
(74) Representative: Gunzelmann, Rainer
(86) International application number: PCT/JP2012/076461
(87) International publication number: WO 2013/054891

(56) References cited:
- EP-A2- 0 971 261
- WO-A1-2004/106857
- JP-A- H09 265 047
- JP-A- 2006 181 021
- US-A- 5 835 133

## Description

### Technical Field

The present invention relates to a 3D endoscope device which forms images for left-eye and right-eye on a single MOS sensor.

### Background Art

A 3D endoscope device which forms images for left-eye and right-eye with parallax on a single MOS sensor to realize a stereoscopic view is known. From the relationship with a mounting space of the endoscopic scope, a system in which images for left-eye and right-eye are formed on a single imaging element with a light receiving surface having a substantially square shape is preferably used compared to a system which uses a plurality of imaging elements.

When forming an image for left-eye and an image for right-eye on a single imaging element, in general, the image for left-eye and the image for right-eye are formed in left and right divided regions of the light receiving surface of the imaging element. However, when obtaining a high-definition image, it is necessary to generate a 16:9 horizontally long image. Accordingly, in the system in which the images are formed in the left and right divided regions of the light receiving surface of the imaging element having a substantially square shape, the image should be enlarged later in the horizontal direction with a large magnification, causing deterioration of image quality. As a means for solving this problem, Patent Document 1 suggests that, as shown in FIG. 7, an image for left-eye and an image for right-eye are formed in upper and lower divided regions S11 and S12 of a light receiving surface of an imaging element.

EP 0 971 261 A2 is the basis for the preamble of claim 1 and discloses a stereoscopic picture obtaining device for obtaining a stereoscopic picture with parallax by a single camera which comprises two objective lenses for simultaneously obtaining two pictures of a subject from different locations of viewpoint, picture merge means for merging the two pictures into a single picture and a CCD pickup device for converting the single image into image data. The two pictures may be merged such that they are arranged in an orthogonal direction to a parallax direction.

US 5 835 133 discloses a camera for generating a stereo video signal which comprises a CCD sensor responsive to an image projected thereon and an optical assembly with first and second optical channels for focusing substantially coincident portions of respective fields of view of the first and second optical channels onto abutting left and right regions of the CCD sensor.

### Citation List

### Prior Art Document

[Patent Document 1] Republished Japanese Translation No. 2004/106857 of the PCT International Publication for Patent Applications

### Summary of the Invention

### Problem to be Solved by the Invention

The technique described in Patent Document 1 is effective when left and right images are formed on a single imaging element in order to obtain a horizontally long high-definition image. However, in Patent Document 1, since the type of imaging element is not specified, there is no description of a problem when the imaging element is a MOS sensor. If a mechanical shutter is not used and a MOS sensor is used with no light blocking period, from the characteristic of a rolling shutter, the time at which optical information is accumulated in pixels as electrical information differs according to the positions of the formed images.

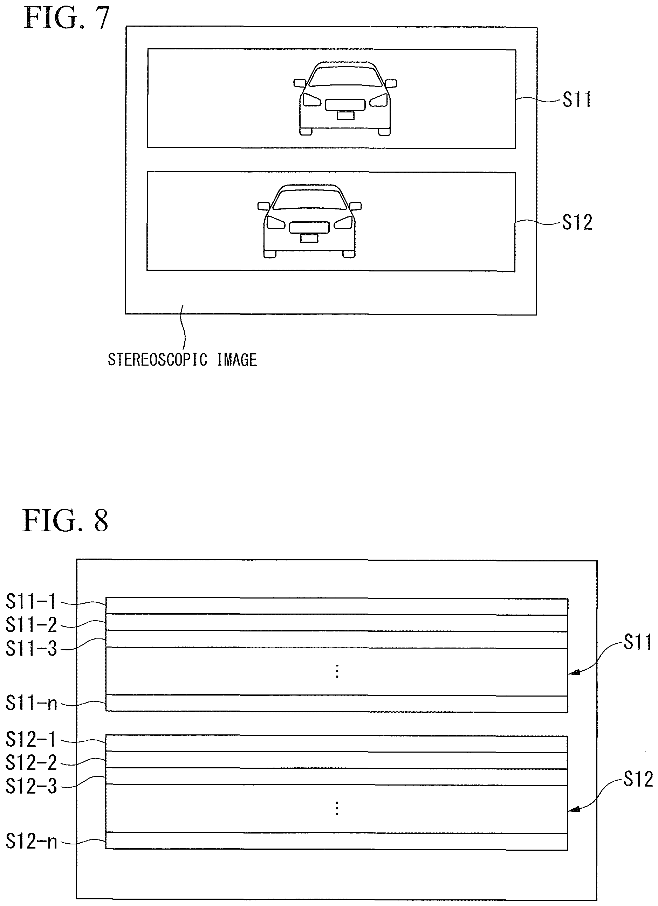

For example, as shown in FIG. 8, in a plurality of divided regions of a region S11, optical information is accumulated in an order of a region S11-1, a region S11-2, a region S11-3, ..., and a region S11-n from the top toward the bottom. After optical information is accumulated in the region S11-n of the region S11, in a plurality of divided regions of a region S12, optical information is accumulated in pixels in an order of a region S12-1, a region S12-2, a region S12-3, ..., and a region S12-n from the top toward the bottom.

For this reason, parallax corresponding to half of one frame period occurs between images at corresponding positions (for example, the region S11-1 and the region S12-1) of the image for left-eye and the image for right-eye, and when imaging a moving subject, positional displacement occurs between the left and right images. Since the 3D endoscope device is a device which allows a subject to be stereoscopically viewed using parallax, positional displacement by a time difference disturbs appropriately set parallax, and stereoscopic image cannot be displayed.

In a 3D endoscope, the arrangement of imaging elements or the configuration of an optical system is determined with high precision in order to obtain appropriate parallax, the endoscopic scope is deformed with the use of autoclave or the like, or water droplets are stuck to a lens upon the use of the endoscope, causing a problem in that physical displacement occurs. Since it is difficult to predict the displacement in advance, there is a need for a function of detecting displacement before the use of the endoscopic scope or during the use of the endoscopic scope if necessary, and electronically correcting the displacement. If displacement over time or according to the operating conditions is added to displacement by the characteristic of the rolling shutter of the MOS sensor, it is not possible to detect only the former displacement to be corrected.

The present invention has been accomplished in consideration of the above-described problem, and an object of the present invention is to provide a 3D endoscope device capable of suppressing displacement between left and right images due to the characteristic of a rolling shutter of a MOS sensor.

### Means for solving the Problem

The present invention has been accomplished in order to solve the above-described problem, and a 3D endoscope device with the features of claim 1 is provided.

In the 3D endoscope device, the MOS sensor may read data by scanning the plurality of first divided regions and the plurality of second divided regions by raster scan, and a direction of the raster scan may be orthogonal to the parallax direction. The image processor may include an image processing unit which performs the image processing, a separation unit which separates the video signal into a video signal for left-eye corresponding to the image for left-eye and a video signal for right-eye corresponding to the image for right-eye, and an adjustment unit which rearranges an order of data constituting each of the video signal for left-eye and the video signal for right-eye so as to be the same as an order of data when data is read by scanning the plurality of first divided regions and the plurality of second divided regions by the raster scan in a same direction as the parallax direction.

The image processor may include a control unit which instructs a calibration operation before a normal operation or during
the normal operation, a displacement detection unit which detects an amount of displacement of the image for left-eye and the image for right-eye during the calibration operation, a correction amount calculation unit which calculates correction amounts of the image for left-eye and the image for right-eye during the calibration operation, and a correction unit which performs correction on the video signal according to the correction amounts of the image for left-eye and the image for right-eye.

The displacement detection unit may detect the amount of displacement for at least one factor of brightness, white balance, size, rotation, and parallel movement, and the correction amount calculation unit may calculate a correction amount corresponding to the amount of displacement for each factor.

### Advantageous Effects of Invention

According to the above description, when reading data constituting the video signal from the first region and the second region, the MOS sensor reads data by alternately scanning the first divided region at a position corresponding to the image for left-eye and the second divided region at a position corresponding to the image for right-eye, whereby it is possible to suppress displacement between the left and right images by the characteristic of the rolling shutter of the MOS sensor.

### Brief Description of Drawings

FIG. 1 is a configuration diagram showing the schematic configuration of a 3D endoscope device according to an embodiment of the present invention.
FIG. 2 is a reference diagram showing a light receiving surface of a CMOS sensor in a 3D endoscope device according to an embodiment of the present invention.
FIG. 3 is a reference diagram showing a form in which a CMOS sensor in a 3D endoscope device according to an embodiment of the present invention reads data constituting a video signal from respective pixels by scanning a light receiving surface.
FIG. 4 is a block diagram showing the configuration of an image processor in a 3D endoscope device according to an embodiment of the present invention.
FIG. 5 is a reference diagram showing a form of processing which is performed by a video signal separation unit and a video signal adjustment unit of an image processor in a 3D endoscope device according to an embodiment of the present invention.
FIG. 6 is a reference diagram showing a form in which a CMOS sensor in a 3D endoscope device according to an embodiment of the present invention reads data constituting a video signal from respective pixels by scanning a light receiving surface.
FIG. 7 is a reference diagram showing a form in which left and right images are formed on an imaging element.
FIG. 8 is a reference diagram illustrating a difference in accumulation time by the characteristic of a rolling shutter of a MOS sensor.

### Description of Embodiments

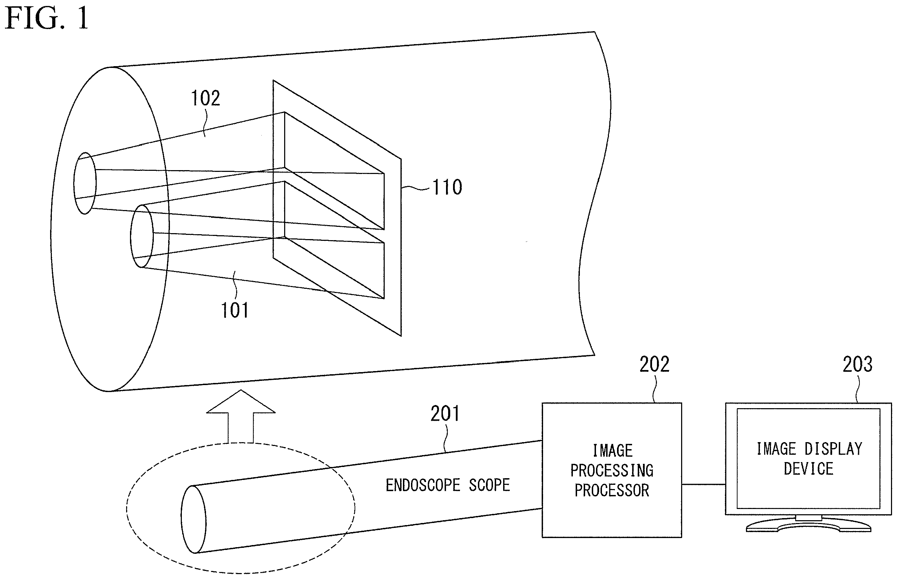

Hereinafter, an embodiment of the present invention will be described referring to the drawings. FIG. 1 shows the schematic configuration of a 3D endoscope device according to an embodiment of the present invention. The outline of the 3D endoscope device will be described with reference to FIG. 1.

The 3D endoscope device includes an endoscopic scope 201 having a left-eye optical system 101, a right-eye optical system 102, and a CMOS sensor 110 (MOS sensor), an image processor 202, and an image display device 203 as a monitor. The left-eye optical system 101, the right-eye optical system 102, and the CMOS sensor 110 are arranged at the distal end of the endoscopic scope 201.

The left-eye optical system 101 and the right-eye optical system 102 are two optical systems which form light corresponding to an image for left-eye and an image for right-eye. The left-eye optical system 101 and the right-eye optical system 102 have an angle of view suitable for a high-definition image, for example, with an aspect ratio of 16:9. The left-eye optical system 101 and the right-eye optical system 102 are arranged in the form of providing parallax appropriate for three-dimensional display between the image for left-eye and the image for right-eye. Two systems of light (first light and second light) having passed through the left-eye optical system 101 and the right-eye optical system 102 are separated vertically on a light receiving surface of the CMOS sensor 110 and formed as an image for left-eye and an image for right-eye.

The CMOS sensor 110 generates a video signal based on the image for left-eye (first image) and the image for right-eye (second image) formed on the light receiving surface. The image processor 202 performs image processing on the video signal output from the CMOS sensor 110. The image display device 203 displays an image including the image for left-eye and the image for right-eye on the basis of the video signal processed by the image processor 202.

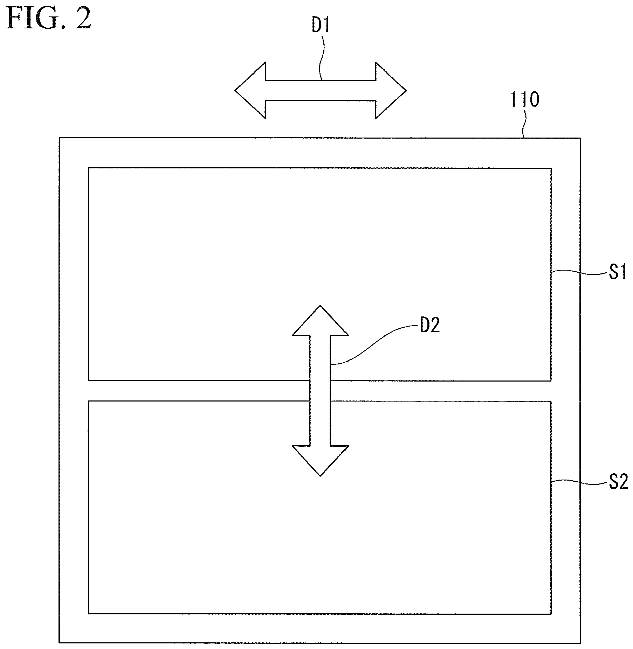

The vertical and horizontal relationship expressed herein will be explained with reference to FIG. 2. FIG. 2 shows the light receiving surface of the CMOS sensor 110. On the light receiving surface of the CMOS sensor 110, a plurality of pixels which generate data based on the formed light are arranged in a matrix. The light receiving surface of the CMOS sensor 110 has a region S1 (first region) where light having passed through the left-eye optical system 101 is formed as an image for left-eye, and a region S2 (second region) where light having passed through the right-eye optical system 102 is formed as an image for right-eye. A direction (parallax direction) in which parallax is provided between the image for left-eye and the image for right-eye is a horizontal direction (the direction of arrow D1 of FIG 2), and the direction (the arrangement direction of the region S1 and the region S2) of a line which connects the centers of the image for left-eye and the image for right-eye formed to be separated in two on the light receiving surface of the CMOS sensor 110 is a vertical direction (the direction of arrow D2 of FIG. 2). The two directions are orthogonal to each other.

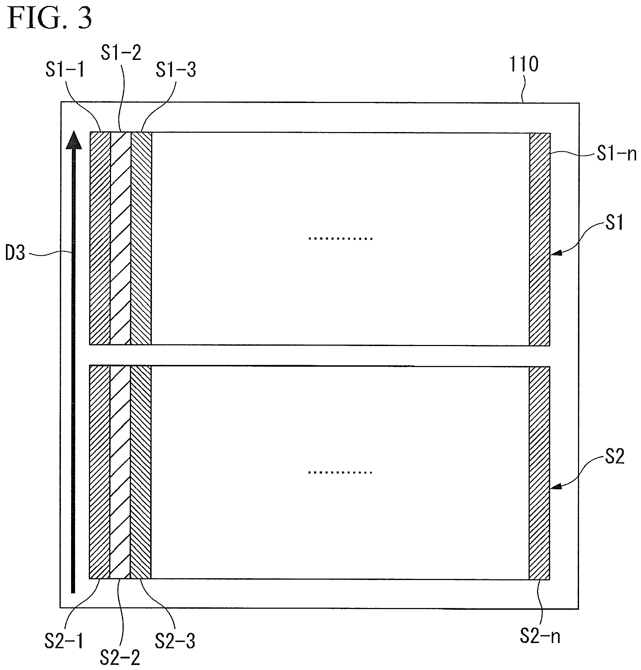

FIG. 3 shows a form in which the CMOS sensor 110 reads data constituting a video signal from the respective pixels arranged in a matrix on the light receiving surface by scanning the light receiving surface by raster scan. The direction (the direction of arrow D3 of FIG. 3) in which the CMOS sensor 110 scans the light receiving surface is orthogonal to the parallax direction. The region S1 and the region S2 are divided into a plurality of divided regions. The region S1 has a divided region S1-1, a divided region S1-2, a divided region S1-3, ..., and a divided region S1-n (first divided regions) which are divided in units of columns of pixels arranged in a matrix. The region S2 has a divided region S2-1, a divided region S2-2, a divided region S2-3, ..., and a divided region S2-n (second divided regions) which are divided in units of columns of pixels arranged in a matrix. Each divided region in the region S1 is associated with each divided region in the same column of the region S2. For example, the divided region S1-1 corresponds to the divided region S2-1, and the divided region S1-n corresponds to the divided region S2-n.

The CMOS sensor 110 scans the light receiving surface in the direction of arrow D3 and reads data constituting the video signal from the respective pixels of each divided region. Accordingly, the respective divided regions in the region S1 and the respective divided regions in the region S2 are alternately scanned. Specifically, the respective divided regions are scanned in an order (sequence) of the divided region S2-1, the divided region S1-1, the divided region S2-2, the divided region S1-2, the divided region S2-3, the divided region S1-3, ..., the divided region S2-n, and the divided region S1-n. In this way, the region S1 and the region S2 are alternately scanned in the same direction with the divided regions divided in units of columns as a scan unit.

Accordingly, the difference in the time (accumulation start time or end time) at which optical information is accumulated as electrical information at the corresponding positions of an image for left-eye and an image for right-eye (the positions in an image for left-eye and an image for right-eye which are identical on the respective images) becomes a very small amount of time which is half the scan time per line. For example, the difference between the time at which optical information is accumulated as electrical information in the uppermost pixel of the divided region S1-1 and the time at which optical information is accumulated as electrical information in the uppermost pixel of the corresponding divided region S2-1 is half the scan time per line (the total scan time of the divided region S1-1 and the divided region S2-1). The CMOS sensor 110 outputs a video signal, in which data of an image for left-eye and data of an image for right-eye are alternately mixed, to the image processor 202.

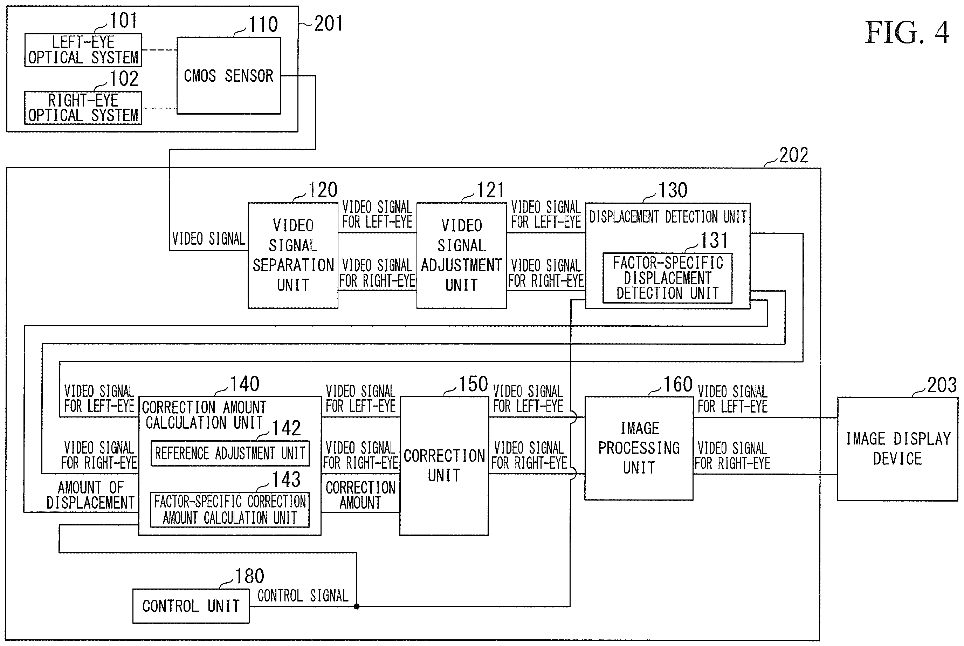

FIG. 4 shows the detailed configuration of the image processor 202. The image processor 202 has a video signal separation unit 120, a video signal adjustment unit 121, a displacement detection unit 130, a correction amount calculation unit 140, a correction unit 150, an image processing unit 160, and a control unit 180.

The video signal separation unit 120 separates the video signal, in which data of the image for left-eye and data of the image for right-eye are alternately mixed, into a video signal for left-eye constituted by data of the image for left-eye and a video signal for right-eye constituted by data of the image for right-eye. Accordingly, subsequent processing can be performed in a unit of each of the images for left-eye and right-eye.

The video signal adjustment unit 121 adjusts the order of data constituting each of the video signal for left-eye and the video signal for right-eye output from the video signal separation unit 120. The light receiving surface of the CMOS sensor 110 is scanned in the vertical direction, whereby the sequence of data of the respective pixels is in a special state. For this reason, the video signal adjustment unit 121 adjusts (rearranges) the order of data constituting the video signal for left-eye so as to be the same as the order of data of the respective pixels when the region S1 is scanned in the same direction as the parallax direction by raster scan. The video signal adjustment unit 121 adjusts (rearranges) the order of data constituting the video signal for right-eye so as to be the same as the order of data of the respective pixels when the region S2 is scanned in the same direction as the parallax direction by raster scan. Accordingly, the order of data constituting each of the video signal for left-eye and the video signal for right-eye is the same as the order of data to be finally input to the image display device 203.

While a memory is generally used for data rearrangement, if a video signal for left-eye and a video signal for right-eye are separately written in a left-eye memory and a right-eye memory, and data is managed in units of left and right memories, it is not necessary to separately prepare a separation process.

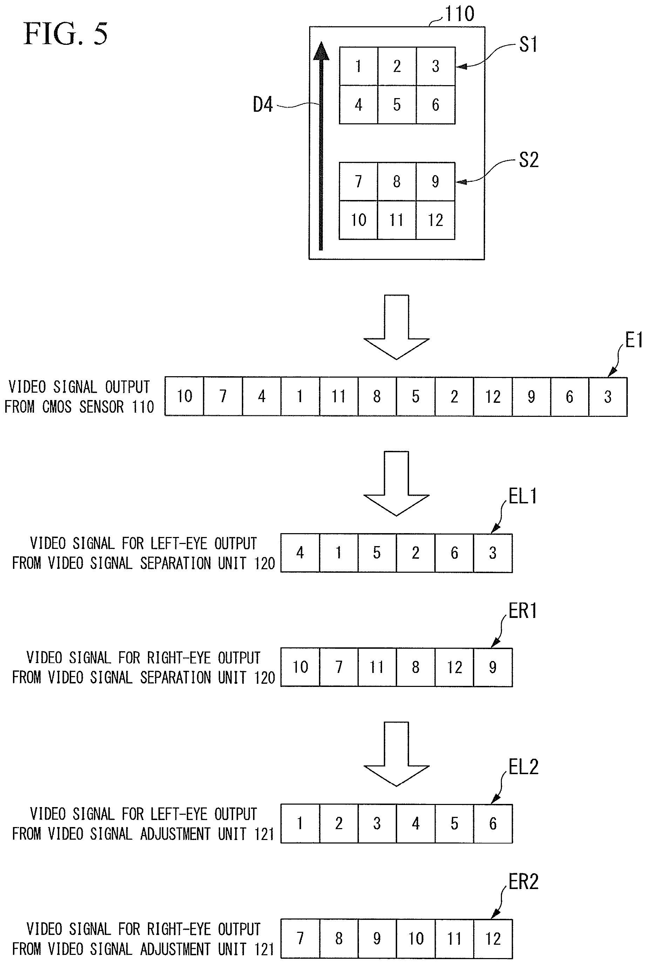

FIG. 5 shows a form of processing which is performed by the video signal separation unit 120 and the video signal adjustment unit 121. For simplification of description, on the light receiving surface of the CMOS sensor 110, the pixels of the region S1 where the image for left-eye is formed and the pixels of the region S2 where the image for right-eye is formed are arranged in two rows and three columns. In order to distinguish between 12 pixels shown in FIG. 5, the numbers of 1 to 12 are attached to the respective pixels.

Since the light receiving surface of the CMOS sensor 110 is scanned in the vertical direction (the direction of arrow D4 of FIG. 5), data of respective pixels in a video signal E1 output from the CMOS sensor 110 are arranged in an order shown in FIG. 5. The video signal separation unit 120 separates the video signal E1 into a video signal for left-eye ELI and a video signal for right-eye ER1. The video signal adjustment unit 121 adjusts the order of data of the respective pixels constituting the video signal for left-eye ELI and generates a video signal for left-eye EL2. The video signal adjustment unit 121 adjusts the order of data of the respective pixels constituting the video signal for right-eye ER1 and generates a video signal for right-eye ER2. The order of data of the respective pixels in the video signal for left-eye EL2 is the same as the order of data of the respective pixels when the region S1 is scanned in the same direction as the parallax direction by raster scan. The order of data of the respective pixels in the video signal for right-eye ER2 is the same as the order of data of the respective pixels when the region S2 is scanned in the same direction as the parallax direction by raster scan.

The displacement detection unit 130 and the correction amount calculation unit 140 operate on the basis of a control signal output from the control unit 180. The control signal output from the control unit 180 is a signal which instructs an operation mode. The 3D endoscope device of this embodiment has a normal mode and a calibration mode as the operation mode. The calibration mode is instructed before the normal operation or during the normal operation. If the control signal instructs the calibration mode, the displacement detection unit 130 and the correction amount calculation unit 140 detect displacement between the image for left-eye and the image for right-eye on the basis of the video signal for left-eye and the video signal for right-eye, and calculate a correction amount. The calculated correction amount is saved at the end of calibration and used in the normal mode. In the normal mode, the displacement detection unit 130 stops operating, or cancels the calculated amount of displacement or does not update the amount of displacement even if operates. In the normal mode, the correction amount calculation unit 140 stops operating, except for below-described strain correction, or cancels the calculated correction amount or does not update the correction amount even if operates. Other than in the displacement detection unit 130 and the correction amount calculation unit 140, a single operation is performed without reference to the control signal.

The displacement detection unit 130 has five factor-specific displacement detection units 131 which individually detect displacement for respective factors of brightness, white balance, size, rotation, and parallel movement. In FIG. 4, only one factor-specific displacement detection unit 131 is shown, and other four factor-specific displacement detection units 131 are omitted. Hereinafter, the operation of the factor-specific displacement detection unit 131 in the calibration mode will be described in detail.

In order to detect displacement, in the calibration mode, the 3D endoscope device images a calibration tool on which a chart image is drawn. Although various images are considered as the chart image drawn on the calibration tool, in this embodiment, an example where a square which is blackened in the central portion of a white background is drawn will be described.

The factor-specific displacement detection unit 131 for brightness detects the amount of displacement of brightness of the image for right-eye with respect to the image for left-eye from the luminance average of the image for left-eye and the image for right-eye or the like. A range in which the average is obtained may be the entire image or just a predefined range. Although the amount of displacement of brightness is the ratio of luminance, a difference in luminance may be used.

In regard to the amount of displacement of white balance, the amount of displacement of the image for left-eye with respect to a balanced state and the amount of displacement of the image for right-eye with respect to a balanced state are detected by the factor-specific displacement detection unit 131 for white balance.

In regard to the amount of displacement of size, rotation, and parallel movement, after predetermined strain correction is performed on the video signal for left-eye and the video signal for right-eye in advance, the amount of displacement is detected. In order to reproduce an image which is preferred for the lens characteristic of the endoscopic scope or an operator, predetermined strain occurs in an endoscope image. It is possible to accurately detect the amount of displacement of size, rotation, and parallel movement by removing the strain.

The factor-specific displacement detection units 131 for size, rotation, and parallel movement analyze the image for left-eye and the image for right-eye to detect the amount of displacement. In a state where strain is removed and the square can be recognized as a square, the boundary position of black and white is detected, whereby the coordinates of the four vertexes of the square are easily obtained.

The factor-specific displacement detection unit 131 for size calculates the ratio of the distances between the vertexes of the respective images, and for example, detects the ratio of the distances between the vertexes of the image for right-eye with respect to the image for left-eye as the amount of displacement. In this embodiment, the distance between the vertexes of each image corresponds to the size of each image. Since the distance between the chart image drawn on the calibration tool and the lens is constant, and a predetermined amount of intrinsically set parallax does not affect the size, it should suffice that simply the ratio of size is obtained. For example, the distance between two arbitrary vertexes among the four vertexes detected from the image for left-eye and the distance between two vertexes corresponding to the two vertexes in the image for left-eye with the distance calculated, among the four vertexes detected from the image for right-eye are calculated, and the ratio of the distances is calculated.

The factor-specific displacement detection unit 131 for rotation calculates an inclination angle obtained from the vertexes of the respective images, and for example, detects the difference in the inclination angle of the image for right-eye with respect to the image for left-eye as the amount of displacement. Since the distance between the chart image drawn on the calibration tool and the lens is constant, and a predetermined amount of intrinsically set parallax does not affect the inclination angle, it should suffice that the difference between simply the inclination angles is obtained. For example, the inclination angle of a line passing through two arbitrary vertexes among the four vertexes detected from the image for left-eye and the inclination angle of a line passing through two vertexes corresponding to the two vertexes in the image for left-eye, through which the line with the inclination angle calculated passes, among the four vertexes detected from the image for right-eye are calculated, and the difference between the inclination angles is calculated.

The factor-specific displacement detection unit 131 for parallel movement calculates the difference between the center positions of the respective images, and for example, detects the difference in the position of the image for right-eye with respect to the image for left-eye as the amount of displacement. Instead of simply using the difference in the position as the amount of displacement, the amount of displacement is obtained taking into consideration a predetermined amount of intrinsically set parallax.

In regard to displacement other than white balance, although a case where the amount of displacement is detected with reference to the image for left-eye has been described, the amount of displacement may be detected with reference to the image for right-eye. The above-described detection method for the amount of displacement is just an example, and various detection methods may be considered.

The correction amount calculation unit 140 has a reference adjustment unit 142 and five factor-specific correction amount calculation units 143 which calculate a correction amount of displacement of each of brightness, white balance, size, rotation, and parallel movement. In FIG. 4, only one factor-specific correction amount calculation unit 143 is shown, and other four factor-specific correction amount calculation units 143 are omitted. Hereinafter, the operation of the factor-specific correction amount calculation unit 143 in the calibration mode will be described in detail.

Although there is an absolute reference for white balance correction, there is no absolute reference for brightness, size, rotation, and parallel movement. Furthermore, although the image for left-eye and the image for right-eye are compared to each other to understand the amount of displacement between both images, it is difficult to understand whether either the image for left-eye or the image for right-eye is displaced or both the image for left-eye and the image for right-eye are displaced.

Accordingly, in order to allow a user to select an image as a reference of brightness, size, inclination angle, and position from the image for left-eye and the image for right-eye, the reference adjustment unit 142 is provided. The reference adjustment unit 142 selects an image instructed by the user as a reference of brightness, size, inclination angle, and position from the image for left-eye and the image for right-eye.

The factor-specific correction amount calculation unit 143 for white balance calculates the correction amounts of the image for left-eye and the image for right-eye on the basis of an absolute amount of displacement of white balance. Specifically, coefficients which are multiplied by the video signal for left-eye and the video signal for right-eye are calculated so as to have a state where white balance is adjusted.

The factor-specific correction amount calculation unit 143 for each of brightness, size, rotation, and parallel movement calculates the correction amount of the other image with reference to one image selected from the image for left-eye and the image for right-eye by the reference adjustment unit 142. Hereinafter, as illustrated as the operation of the correction amount calculation unit 140, a calculation method for a correction amount and a correction method when the relative amount of displacement of brightness, size, inclination angle, and position of the image for right-eye is detected with reference to the image for left-eye will be described.

First, a calculation method for a correction amount and a correction method relating to brightness will be described. Since the ratio of brightness of the image for right-eye with reference to the image for left-eye is detected by the correction amount calculation unit 140, if the reference adjustment unit 142 selects the image for left-eye as a reference, the reciprocal of the ratio of brightness becomes a correction amount. The factor-specific correction amount calculation unit 143 multiplies the respective pixel values of the video signal for right-eye by the correction amount to match the image for right-eye with the image for left-eye. If the reference adjustment unit 142 selects the image for right-eye as a reference, the ratio of brightness becomes a correction amount, and the factor-specific correction amount calculation unit 143 multiplies the respective pixel values of the video signal for left-eye by the correction amount to match the image for left-eye with the image for right-eye.

Next, a calculation method for a correction amount and a correction method relating to size will be described. Since the ratio of size of the image for right-eye with reference to the image for left-eye is detected by the correction amount calculation unit 140, if the reference adjustment unit 142 selects the image for left-eye as a reference, the reciprocal of the ratio of size becomes a correction amount. The factor-specific correction amount calculation unit 143 performs enlargement processing on the video signal for right-eye on the basis of the correction amount to match the image for right-eye with the image for left-eye. If the reference adjustment unit 142 selects the image for right-eye as a reference, the ratio of size becomes a correction amount, and the factor-specific correction amount calculation unit 143 performs enlargement processing on the video signal for left-eye on the basis of the correction amount to match the image for left-eye with the image for right-eye.

Next, a calculation method for a correction amount and a correction method relating to an inclination angle will be described. Since the difference in the inclination angle of the image for right-eye with reference to the image for left-eye is detected by the correction amount calculation unit 140, if the reference adjustment unit 142 selects the image for left-eye as a reference, a value which is -1 times the difference in the inclination angle becomes a correction amount. The factor-specific correction amount calculation unit 143 performs rotation processing on the video signal for right-eye on the basis of the correction amount to match the image for right-eye with the image for left-eye. If the reference adjustment unit 142 selects the image for right-eye as a reference, the difference in the inclination angle becomes a correction amount, and the factor-specific correction amount calculation unit 143 performs rotation processing on the video signal for left-eye on the basis of the correction amount to match the image for left-eye with the image for right-eye.

Next, a calculation method for a correction amount and a correction method relating to a position will be described. Since the difference in the position of the image for right-eye with reference to the image for left-eye is detected by the correction amount calculation unit 140, if the reference adjustment unit 142 selects the image for left-eye as a reference, a value which is -1 times the difference in the position becomes a correction amount. The factor-specific correction amount calculation unit 143 performs parallel movement processing on the video signal for right-eye on the basis of the correction amount to match the image for right-eye with the image for left-eye. If the reference adjustment unit 142 selects the image for right-eye as a reference, the difference in the position becomes a correction amount, and the factor-specific correction amount calculation unit 143 performs parallel movement processing on the video signal for left-eye on the basis of the correction amount to match the image for left-eye with the image for right-eye.

The correction amount calculation unit 140 outputs the calculated correction amount and the video signal for left-eye and the video signal for right-eye which are subjected to predetermined strain correction so as to remove strain in advance. The correction unit 150 corrects the video signal for left-eye and the video signal for right-eye on the basis of the correction amount calculated by the correction amount calculation unit 140.

The correction unit 150 performs gain multiplication in terms of brightness, performs white balance matrix multiplication in terms of white balance, performs zooming processing in terms of size, performs rotation processing in terms of rotation, and performs parallel movement processing (position conversion) in terms of parallel movement. The video signal for left-eye and the video signal for right-eye which are processed by the correction unit 150 are video signals in which strain in the image is removed by strain correction. For this reason, the correction unit 150 performs processing for restoring intrinsic strain on the video signal for left-eye and the video signal for right-eye after correction is performed. The restoration processing is adjusted so as to perform reverse conversion when strain is removed.

The video signal for left-eye and the video signal for right-eye in which displacement other than strain is corrected are subjected to predetermined image processing (image processing for display, such as pixel number conversion, edge correction, or color adjustment) in the image processing unit 160 and are output to the image display device 203 as a monitor. The image display device 203 displays an image including the image for right-eye and the image for left-eye on the basis of the video signal for left-eye and the video signal for right-eye subjected to the image processing by the image processing unit 160.

In the above-described configuration, the displacement detection unit 130, the correction amount calculation unit 140, the correction unit 150, the control unit 180, and parts included in these units are a portion which detects and corrects displacement over time or according to the operating conditions. If this displacement is negligible, these parts are not required.

In a device in which these parts are omitted, the video signal separation unit 120 and the video signal adjustment unit 121 are not necessarily arranged in front of the image processing unit 160, and may be arranged at the back of the image processing unit 160 insofar as the image processing unit 160 performs predetermined image processing in a state where the video signal for left-eye and the video signal for right-eye are mixed.

Next, a modification example of this embodiment will be described. In the above description, although the arrangement of the CMOS sensor is contrived such that the difference in the time at which optical information is accumulated as electrical information is small at the corresponding positions of the image for left-eye and the image for right-eye, a feature in that random access to the CMOS sensor is possible may be used. In this case, addresses are generated such that the access timings at the corresponding positions of the image for left-eye and the image for right-eye become close to each other, and the CMOS sensor scans the light receiving surface according to the generated addresses.

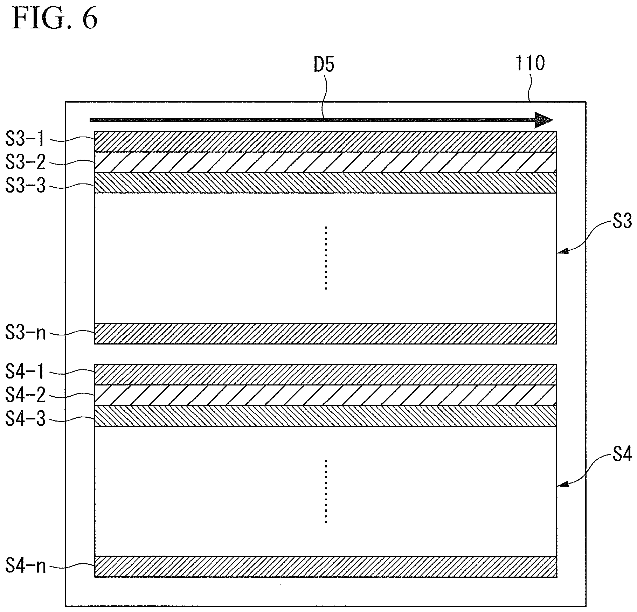

For example, the CMOS sensor 110 may scan the light receiving surface as shown in FIG. 6. FIG. 6 shows a form in which the CMOS sensor 110 scans the light receiving surface by raster scan and reads data constituting a video signal from the respective pixels arranged in a matrix on the light receiving surface. The direction (the direction of arrow D5 of FIG. 6) in which the CMOS sensor 110 scans the light receiving surface is parallel to the parallax direction. A region S3 (first region) and a region S4 (second region) are divided into a plurality of divided regions. The region S3 has a divided region S3-1, a divided region S3-2, a divided region S3-3, ..., and a divided region S3-n (first divided regions) which are divided in units of rows of pixels arranged in a matrix. The region S4 has a divided region S4-1, a divided region S4-2, a divided region S4-3, ..., and a divided region S4-n (second divided region) which are divided in units of rows of pixels arranged in a matrix. Each divided region in the region S3 is associated with each divided region of a corresponding row in the region S4. For example, the divided region S3-1 corresponds to the divided region S4-1, and the divided region S3-n corresponds to the divided region S4-n.

The CMOS sensor 110 scans the light receiving surface in the direction of arrow D5 and reads data constituting a video signal from the respective pixels of each divided region. Accordingly, the respective divided regions in the region S3 and the respective divided regions in the region S4 are alternately scanned. Specifically, the respective divided regions are scanned in an order (sequence) of the divided region S3-1, the divided region S4-1, the divided region S3-2, the divided region S4-2, the divided region S3-3, the divided region S4-3, ..., the divided region S3-n, and the divided region S4-n. In this way, the region S3 and the region S4 are alternately scanned in the same direction with the divided regions divided in units of rows as a scan unit.

Accordingly, the difference in the time (accumulation start time or end time) at which optical information is accumulated as electrical information at the corresponding positions of an image for left-eye and an image for right-eye (the positions in an image for left-eye and an image for right-eye which are identical on the respective images) becomes equal to the scan time per line. For example, the difference between the time at which optical information is accumulated as electrical information in the leftmost pixel of the divided region S3-1 and the time at which optical information is accumulated as electrical information in the leftmost pixel of the corresponding divided region S4-1 is the same as the scan time per line (the scan time of each of the divided region S1-1 and divided region S2-1).

As described above, according to this embodiment, when reading data constituting a video signal from the first region where the image for left-eye is formed and the second region where the image for right-eye is formed, the CMOS sensor 110 reads data by alternately scanning the divided region corresponding to the image for left-eye and the divided region at a position corresponding to the image for right-eye, whereby it is possible to make the difference in the time (accumulation start time or end time), at which optical information is accumulated as electrical information, small at the corresponding positions of the image for left-eye and the image for right-eye. For this reason, it is possible to suppress displacement between the left and right images by the characteristic of the rolling shutter. Therefore, an appropriate video signal which is displayed as a high-definition image is obtained, and even if a moving subject is imaged, it is possible to suppress the influence of a time difference between the left and right images.

When the CMOS sensor 110 reads data by scanning a plurality of divided regions by raster scan, since the direction of raster scan is orthogonal to the parallax direction, the difference in the time (accumulation start time or end time), at which optical information is accumulated as electrical information, at the corresponding positions of the image for left-eye and the image for right-eye, is half the scan time per line. For this reason, even if a moving subject is imaged, it is possible to suppress the influence of a time difference between the left and right images.

The video signal separation unit 120 separates the video signal output from the CMOS sensor 110 into a video signal for left-eye and a video signal for right-eye, and the video signal adjustment unit 121 rearranges the order of data constituting each of the video signal for left-eye and the video signal for right-eye so as to be the same as the order of data when the divided regions are scanned in the same direction as the parallax direction by raster scan. Accordingly, even if the arrangement of data in the video signal output from the CMOS sensor 110 is in a special state, it is possible to generate a video signal for left-eye and a video signal for right-eye corresponding to an input format of a normal image display device.

The displacement detection unit 130 detects the amounts of displacement of the image for left-eye and the image for right-eye during the calibration operation, the correction amount calculation unit 140 calculates the correction amounts of the image for left-eye and the image for right-eye during the calibration operation, and the correction unit 150 corrects the video signal according to the correction amounts of the image for left-eye and the image for right-eye, whereby it is possible to correct displacement that is caused by changing over time or the operating conditions. Therefore, it is possible to constantly generate an image for left-eye and an image for right-eye with appropriate parallax, thereby realizing a stereoscopic view.

The displacement detection unit 130 has the factor-specific displacement detection units 131 which detect the amount of displacement for each factor of brightness, white balance, size, rotation, and parallel movement, and the correction amount calculation unit 140 has the factor-specific correction amount calculation units 143 which calculates the correction amount corresponding to the amount of displacement for each type of displacement. Therefore, even if various kinds of displacement occur in a combined manner, it is possible to detect the amount of displacement separately for each type of displacement and to correct each type of displacement.

### Industrial Applicability

According to the above description, when reading data constituting a video signal from the first region and the second region, the MOS sensor reads data by alternately scanning the divided region at a position corresponding to the image for left-eye and the divided region at a position corresponding to the image for right-eye, whereby it is possible to suppress displacement between the left and right images by the characteristic of the rolling shutter of the MOS sensor.

### Reference Signs List

- 101:: left-eye optical system
- 102:: right-eye optical system
- 110:: CMOS sensor
- 120:: video signal separation unit
- 121:: video signal adjustment unit
- 130:: displacement detection unit
- 131:: factor-specific displacement detection unit
- 140:: correction amount calculation unit
- 142:: reference adjustment unit
- 143:: factor-specific correction amount calculation unit
- 150:: correction unit
- 160:: image processing unit
- 180:: control unit
- 201:: endoscopic scope
- 202:: image processor
- 203:: image display device

## Claims

1. A 3D endoscope device adapted to acquire an image for left-eye and an image for right-eye with parallax, the 3D endoscope device comprising:
an endoscopic scope (201) which has two optical systems (101, 102) that are adapted to form light corresponding to the image for left-eye and the image for right-eye, and a sensor (110) adapted such that a first light and a second light obtained through the two optical systems (101, 102) are formed separately on a single light receiving surface and adapted to generate a video signal based on formed first and second images;
an image processor (202) adapted to perform image processing on the video
signal; and
an image display device (203) adapted to display an image including the image for left-eye and the image for right-eye based on the video signal processed by the image processor (202), **characterized in that**
the sensor (110) is a MOS sensor,
wherein a line which connects a center of the first image and a center of the second image formed on the light receiving surface of the MOS sensor (110) is orthogonal to a parallax direction,
on the light receiving surface of the MOS sensor (110), a first region (S1, S3) where the first image is formed is divided into a plurality of first divided regions, and a second region (S2, S4) where the second image is formed is divided into a plurality of second divided regions, and
the MOS sensor is adapted to read, when reading data constituting the video signal from the first region and the second region, the data by alternately scanning a position where the each first divided region corresponding to the image for left-eye and the each second divided region corresponding to the image for right-eye are corresponding.

2. The 3D endoscope device according to claim 1,
wherein the MOS sensor (110) is adapted to read data by scanning the plurality of first divided regions and the plurality of second divided regions by raster scan, and
a direction of the raster scan is orthogonal to the parallax direction.

3. The 3D endoscope device according to claim 2,
wherein the image processor (202) includes
an image processing unit (160) adapted to perform the image processing,
a separation unit (120) adapted to separate the video signal into a video signal for left-eye corresponding to the image for left-eye and a video signal for right-eye corresponding to the image for right-eye, and
an adjustment unit (121) adapted to rearrange an order of data constituting each of the video signal for left-eye and the video signal for right-eye so as to be the same as an order of data when data is read by scanning the plurality of first divided regions and the plurality of second divided regions by the raster scan in a same direction as the parallax direction.

4. The 3D endoscope device according to claim 3,
wherein the image processor (202) includes
a control unit (180) adapted to instruct a calibration operation before a normal operation or during the normal operation,
a displacement detection unit (130) adapted to detect an amount of displacement of the image for left-eye and the image for right-eye during the calibration operation,
a correction amount calculation unit (140) adapted to calculate correction amounts of the image for left-eye and the image for right-eye during the calibration operation, and
a correction unit (150) adapted to perform correction on the video signal according to the correction amounts of the image for left-eye and the image for right-eye.

5. The 3D endoscope device according to claim 4,
wherein the displacement detection unit (130) is adapted to detect the amount of displacement for at least one factor of brightness, white balance, size, rotation, and parallel movement, and
the correction amount calculation unit (140) is adapted to calculate a correction amount corresponding to the amount of displacement for each factor.

## Patentansprüche

1. 3D-Endoskopvorichtung, die dazu angepasst ist, ein Bild für ein linkes Auge und ein Bild für ein rechtes Auge mit Parallaxe aufzunehmen, wobei die 3D-Endoskopvorrichtung umfasst:
ein Endoskop (201), das zwei optische Systeme (101, 102) aufweist, die dazu angepasst sind, Licht zu bilden, das dem Bild für das linke Auge und dem Bild für das rechte Auge entspricht, und einen Sensor (110), der derart angepasst ist, dass ein erstes Licht und ein zweites Licht, die durch die zwei optischen Systeme (101, 102) erhalten wurden, separat auf einer einzelnen lichtaufnehmenden Oberfläche gebildet werden, und dazu angepasst ist, ein Videosignal basierend auf den gebildeten ersten und zweiten Bildern zu erzeugen;
einen Bildprozessor (202), der dazu angepasst ist, Bildverarbeitung des Videosignals durchzuführen; und
eine Bildanzeigevorrichtung (203), die dazu angepasst ist, ein Bild, das das Bild für das linke Auge und das Bild für das rechte Auge umfasst, basierend auf dem von dem Bildprozessor (202) verarbeiteten Videosignal anzuzeigen, **dadurch gekennzeichnet, dass**
der Sensor (110) ein MOS-Sensor ist,
wobei eine Linie, die eine Mitte des ersten Bildes und eine Mitte des zweiten Bildes verbindet, die auf der lichtaufnehmenden Oberfläche des MOS-Sensors (110) gebildet wurden, orthogonal zu einer Parallaxenrichtung ist,
auf der lichtaufnehmenden Oberfläche des MOS-Sensors (110) eine erste Region (S1, S3), in der das erste Bild gebildet wird, in eine Vielzahl von ersten geteilten Regionen geteilt wird, und eine zweite Region (S2, S4), in der das zweite Bild gebildet wird, in eine Vielzahl von zweiten geteilten Regionen geteilt wird, und
der MOS-Sensor dazu angepasst ist, beim Lesen der Daten, die das Videosignal bilden, aus der ersten Region und der zweiten Region, die Daten durch abwechselndes Abtasten einer Position zu lesen, in der jede erste geteilte Region, die dem Bild für das linke Auge entspricht, und jede zweite geteilte Region, die dem Bild für das rechte Auge entspricht, einander entsprechen.

2. 3D-Endoskopvorrichtung nach Anspruch 1,
wobei der MOS-Sensor (110) dazu angepasst ist, die Daten durch Abtasten der Vielzahl von ersten geteilten Regionen und der Vielzahl von zweiten geteilten Regionen durch Rasterabtastung zu lesen, und
eine Richtung der Rasterabtastung orthogonal zu der Parallaxenrichtung ist.

3. 3D-Endoskopvorrichtung nach Anspruch 2,
wobei der Bildprozessor (202) umfasst
eine Bildverarbeitungseinheit (160), die dazu angepasst ist, die Bildverarbeitung durchzuführen,
eine Trenneinheit (120), die dazu angepasst ist, das Videosignal in ein Videosignal für das linke Auge, das dem Bild für das linke Auge entspricht, und ein Videosignal für das rechte Auge, das dem Bild für das rechte Auge entspricht, zu trennen, und
eine Anpassungseinheit (121), die dazu angepasst ist, eine Reihenfolge der Daten, die jedes von dem Videosignal für das linke Auge und dem Videosignal für das rechte Auge bilden, derart neu anzuordnen, dass sie mit einer Reihenfolge der Daten übereinstimmt, wenn die Daten durch Abtasten der Vielzahl der ersten geteilten Regionen und der Vielzahl der zweiten geteilten Regionen durch die Rasterabtastung in einer gleichen Richtung wie der Parallaxenrichtung gelesen wird.

4. 3D-Endoskopvorrichtung nach Anspruch 3,
wobei der Bildprozessor (202) umfasst
eine Steuerungseinheit (180), die dazu angepasst ist, einen Kalibrierungsvorgang vor einem normalen Vorgang oder während des normalen Vorgangs anzuweisen,
eine Verlagerungserfassungseinheit (130), die dazu angepasst ist, einen Verlagerungsbetrag des Bildes für das linke Auge und des Bildes für das rechte Auge während des Kalibrierungsvorgangs zu erfassen,
eine Korrekturbetragberechnungseinheit (140), die dazu angepasst ist, Korrekturbeträge des Bildes für das linke Auge und des Bildes für das rechte Auge während des Kalibrierungsvorgangs zu berechnen,
eine Korrektureinheit (150), die dazu angepasst ist, eine Korrektur des Videosignals entsprechend der Korrekturbeträge des Bildes für das linke Auge und des Bildes für das rechte Auge durchzuführen.

5. 3D-Endoskopvorrichtung nach Anspruch 4,
wobei die Verlagerungserfassungseinheit (130) dazu angepasst ist, den Verlagerungsbetrag für mindestens einen Faktor von Helligkeit, Weißabgleich, Größe, Rotation und Parallelbewegung zu erfassen, und
die Korrekturbetragberechnungseinheit (140) dazu angepasst ist, einen Korrekturbetrag zu berechnen, der dem Verlagerungsbetrag für jeden Faktor entspricht.

## Revendications

1. Dispositif d'endoscope 3D apte à acquérir une image pour oeil gauche et une image pour oeil droit avec parallaxe, le dispositif d'endoscope 3D comprenant :
un scope endoscopique (201) qui a deux systèmes optiques (101, 102) qui sont aptes à former de la lumière correspondant à l'image pour oeil gauche et à l'image pour oeil droit, et un capteur (110) conçu de telle sorte qu'une première lumière et une seconde lumière obtenues par l'intermédiaire des deux systèmes optiques (101, 102) sont formées séparément sur une unique surface de réception de lumière et apte à générer un signal vidéo sur la base de première et seconde images formées ;
un processeur d'image (202) apte à réaliser un traitement d'image sur le signal vidéo ; et
un dispositif d'affichage d'image (203) apte à afficher une image comprenant l'image pour oeil gauche et l'image pour oeil droit sur la base du signal vidéo traité par le processeur d'image (202), **caractérisé par le fait que**
le capteur (110) est un capteur MOS,
une ligne qui relie un centre de la première image et un centre de la seconde image formées sur la surface de réception de lumière du capteur MOS (110) étant orthogonale à une direction de parallaxe,
sur la surface de réception de lumière du capteur MOS (110), une première région (S1, S3), dans laquelle la première image est formée, étant divisée en une pluralité de premières régions divisées et une seconde région (S2, S4), dans laquelle la seconde image est formée, étant divisée en une pluralité de secondes régions divisées, et
le capteur MOS étant apte à lire, lors de la lecture de données constituant le signal vidéo à partir de la première région et de la seconde région, les données par balayage alterné d'une position dans laquelle chaque première région divisée correspondant à l'image pour oeil gauche et chaque seconde région divisée correspondant à l'image pour oeil droit correspondent l'une à l'autre.

2. Dispositif d'endoscope 3D selon la revendication 1, dans lequel
le capteur MOS (110) est apte à lire des données par balayage de la pluralité de premières régions divisées et la pluralité de secondes régions divisées par balayage tramé, et
une direction du balayage tramé est orthogonale à la direction de parallaxe.

3. Dispositif d'endoscope 3D selon la revendication 2, dans lequel le processeur d'image (202) comprend
une unité de traitement d'image (160) apte à réaliser le traitement d'image,
une unité de séparation (120) apte à séparer le signal vidéo en un signal vidéo pour oeil gauche correspondant à l'image pour oeil gauche et un signal vidéo pour oeil droit correspondant à l'image pour oeil droit, et
une unité d'ajustement (121) apte à réorganiser un ordre de données constituant chacun du signal vidéo pour oeil gauche et du signal vidéo pour oeil droit de façon à être identique à un ordre de données lorsque des données sont lues par balayage de la pluralité de premières régions divisées et de la pluralité de secondes régions divisées par le balayage tramé dans une même direction que la direction de parallaxe.

4. Dispositif d'endoscope 3D selon la revendication 3, dans lequel le processeur d'image (202) comprend
une unité de commande (180) apte à ordonner une opération d'étalonnage avant un fonctionnement normal ou pendant le fonctionnement normal,
une unité de détection de déplacement (130) apte à détecter une valeur de déplacement de l'image pour oeil gauche et de l'image pour oeil droit pendant l'opération d'étalonnage,
une unité de calcul de valeur de correction (140) apte à calculer des valeurs de correction de l'image pour oeil gauche et de l'image pour oeil droit pendant l'opération d'étalonnage, et
une unité de correction (150) apte à réaliser une correction sur le signal vidéo en fonction des valeurs de correction de l'image pour oeil gauche et de l'image pour oeil droit.

5. Dispositif d'endoscope 3D selon la revendication 4, dans lequel
l'unité de détection de déplacement (130) est apte à détecter la valeur de déplacement pour au moins un facteur de luminosité, d'équilibre des blancs, de taille, de rotation et de mouvement parallèle, et
l'unité de calcul de valeur de correction (140) est apte à calculer une valeur de correction correspondant à la valeur de déplacement pour chaque facteur.
